# FASCICULE DE BREVET EUROPEEN

(11) **EP 3 302 414 B1**
(45) Date de publication et mention de la délivrance du brevet: **13.02.2019**
(21) Numéro de dépôt: 16733163.6
(22) Date de dépôt: 02.06.2016
(51) Int. Cl.: A61K 8/37, A61Q 3/02, A61K 8/73, A61K 8/81, A61K 8/85

(54) **COMPOSITION DE VERNIS À ONGLES À SÉCHAGE PHYSIQUE, SON PROCÉDÉ D'APPLICATION ET KIT COMPRENANT UNE TELLE COMPOSITION**
PHYSIKALISCH TROCKNENDE NAGELLACKZUSAMMENSETZUNG, ANWENDUNGSVERFAHREN DAFÜR UND KIT MIT SOLCH EINER ZUSAMMENSETZUNG
PHYSICALLY DRYING NAIL POLISH COMPOSITION, THE APPLICATION METHOD THEREOF, AND KIT INCLUDING SUCH A COMPOSITION

(30) Priorité: 04.06.2015 FR 1555081
(43) Date de publication de la demande: 11.04.2018
(73) Titulaire: Fiabila, 28130 Maintenon (FR)
(72) Inventeur: NOUGUEREDE, Olivier, 28130 Hanches (FR); LEGOUPY, Anne-Lise, 28360 Prunay Le Gillon (FR)
(74) Mandataire: Le Cloirec, Claudine
(86) Numéro de dépôt international: PCT/FR2016/051315
(87) Numéro de publication internationale: WO 2016/193625

(56) Documents cités:
- EP-A1- 1 595 524
- EP-A1- 2 762 126
- WO-A1-98/48769
- WO-A1-99/55290
- WO-A1-2013/106222
- WO-A2-02/03913
- GB-A- 2 496 990
- US-A1- 2003 012 750
- Anonymous: "Radcure UV/EB energy curable resins", 2014, Allnex SA, XP002751168, page 20

## Description

La présente invention concerne le domaine des vernis à ongles.

Les vernis à ongles peuvent être classés actuellement en deux grandes catégories :
On connait, d'une part, les vernis à ongles à base nitrocellulose diluée par des solvants organiques, contenant aussi un plastifiant, une ou plusieurs résines et un agent thixotrope dans le cas des produits colorés. Ces vernis sont faciles à appliquer en une ou plusieurs couches minces sur les ongles. Le séchage par évaporation du solvant conduit en quelques minutes à la création d'un film qui adhère à l'ongle et durcit. Le démaquillage s'effectue par application d'un coton imbibé d'une solution dissolvante (acétate d'éthyle, ....) qui solubilise le vernis pour l'éliminer totalement de l'ongle. Ces vernis présentent une faible tenue de la brillance, qui diminue après quatre jours en moyenne, ainsi qu'une faible résistance à l'usure : selon l'activité de la personne, le vernis solvanté appliqué sur les ongles doit donc être renouvelé au bout de quelques jours, une semaine au plus.

Pour augmenter la résistance et la durée du vernis sur l'ongle, ont été proposés d'autre part des vernis dénommés « vernis gels ». La composition de base renferme, outre le(s) polymère(s) filmogène(s) à base de cellulose, des monomères et/ou oligomères de faible masse molaire, polymérisables par UV. Le durcissement du vernis exige donc, après application du vernis gel sur les ongles, l'exposition de ceux-ci pendant quelques minutes sous une lampe à rayons ultra-violets, en vue de réaliser la polymérisation/réticulation. Une fois réticulés, ces polymères confèrent au vernis une plus grande résistance et donc une plus longue durée sur l'ongle. Ce type de vernis-gel très résistant, peut alors rester sur l'ongle au moins deux semaines sans être endommagé. Cependant la pousse de l'ongle implique de renouveler leur application au-delà de ce délai. La résistance améliorée des vernis-gels par rapport aux vernis-solvants présente cependant un inconvénient majeur : ces vernis-gels sont beaucoup plus difficiles à démaquiller : alors que pour retirer un vernis solvant un simple passage d'un coton imbibé de solvant suffit, la dissolution d'un vernis gel exige le contact, voire le trempage direct, de l'ongle avec un dissolvant (très souvent à base d'acétone) pendant au moins dix minutes. Ce contact prolongé avec un dissolvant est en outre traumatisant pour l'ongle. Un temps de pose plus long, la nécessité de disposer d'une lampe UV lors de l'application, et un démaquillage long, fastidieux et traumatisant pour les ongles découragent les utilisateurs d'appliquer de tels vernis gels.

De plus la présence d'agents de réticulation tels que des photo-initiateurs dans la composition de vernis induit deux autres inconvénients : d'une part la présence de ces photo-initiateurs implique, pour éviter une polymérisation lors du stockage, que le vernis soit conditionné dans des flacons opaques, ce qui nuit à l'attractivité pour le client qui souhaite en visualiser la couleur avant achat ou utilisation, et d'autre part l'ongle est mis en contact direct avec les photo-initiateurs qui sont généralement des molécules sensibilisantes, allergisantes et parfois toxiques.

Il subsiste donc un réel besoin d'un vernis présentant les avantages à la fois des vernis solvants et des vernis gels, sans leurs inconvénients respectifs. La présente invention a pour objectif principal de pallier les inconvénients de ces deux catégories actuelles de vernis à ongles.

Un premier but de l'invention est donc de proposer une composition de vernis apte à être réticulée sans exposition à une lampe UV.

Un autre but de l'invention est de proposer une composition de vernis présentant, après application sur l'ongle, une tenue améliorée, notamment une meilleure résistance à l'usure et une tenue de la brillance améliorée par rapport à un vernis solvanté.

Un autre but de l'invention est de proposer une composition de vernis présentant un temps de maquillage identique et un démaquillage équivalent à celui d'un vernis solvanté.

Un autre but de l'invention est de proposer une composition de vernis ne renfermant pas de substances sensibilisantes ou toxiques venant au contact de l'ongle.

A cet effet la composition de vernis pour les ongles selon l'invention, renfermant au moins un agent filmogène, un plastifiant et un solvant, est caractérisée en ce qu'elle comprend également un ou plusieurs oligomère(s) de masse molaire supérieure à 1500 g/mol et/ou dont la température de transition vitreuse Tg est supérieure à 25 °C, ledit oligomère possédant des doubles liaisons acryliques ou méthacryliques disponibles pour réagir avec des radicaux, sans renfermer d'agent de réticulation de l'oligomère, tel qu'un photo-initiateur.

De tels oligomères présentent la propriété d'un séchage « physique », c'est-à-dire qu'après quelques minutes, le vernis appliqué sur l'ongle est sec, ne présentant plus un caractère collant (tack), au contraire du comportement des vernis gels de l'art antérieur. Plus particulièrement, l'oligomère présente une dureté Persoz d'au moins 60 s, de préférence d'au moins 80 s, de préférence encore d'au moins 100 s, lorsqu'il est appliqué sur une plaque de verre selon une épaisseur de 100 µm humide (correpondant à 50 µm sec), puis séché24 h à 25°C à l'abri de la lumière.

De manière avantageuse, la masse molaire de l'oligomère est supérieure ou égale à 2000 g/mol, de préférence supérieure ou égale à 3000 g/mol.

Ledit oligomère présente avantageusement une température de transition vitreuse Tg supérieure à 30 °C, de préférence supérieure à 35 °C, de préférence encore supérieure à 40°C. Cette température de transition vitreuse Tg est mesurée sur l'oligomère brut avant toute réticulation.

Il s'est avéré que les oligomères de type (méth)acrylate possédant donc des doubles liaisons acryliques ou méthacryliques disponibles pour réagir avec des radicaux, en l'absence de photo-initiateur et satisfaisant aux conditions ci-dessus, permettent d'obtenir les propriétés de brillance améliorée, une meilleure résistance à l'usure, tout en présentant un temps de maquillage identique et un démaquillage équivalent à celui d'un vernis solvanté.

Les oligomères de type (méth)acrylate non aromatiques sont préférés, les oligomères renfermant des noyaux aromatiques ayant une tendance au jaunissement.

L'oligomère de type (méth)acrylate est de préférence choisi parmi : un poly(méth)acrylate (méth)acrylé, un polyester (méth)acrylé, un polyéther (méth)acrylé, un uréthane (méth)acrylé, un polyuréthane (méth)acrylé ou un époxy (méth)acrylé ou un mélange de ceux-ci.

Des exemples de tels (méth)acrylates pouvant être utilisés dans les compositions selon l'invention sont les produits :
Ebecryl 1200 de la société Allnex : poly(méth)acrylate acrylé,
Ebecryl 4654 de la société Allnex : polyuréthane acrylé
Doublemer 347-1 : de la société Double Bound : polyacrylate modifié silane acrylé
Lumicryl U721 de la société Estron : poly(méth)acrylate acrylé

Leurs propriétés sont présentées dans le tableau 1 ci-après.

**Tableau 1**

| **Oligomère** | **Nature** | **Masse molaire (g/mol)** | **Tg (°C)** | **Dureté Persoz (s)** |
|---|---|---|---|---|
| Ebecryl 1200 | poly(méth)acrylate acrylé | > 10.000 | 65 | 90 |
| Ebecryl 4654 | Polyuréthane acrylé | 15.000 | 92 | 210 |
| Doublemer 347-1 | Polyacrylate modifié silane acrylé | > 12.000 | 43 | 150 |
| Lumicryl U 721 | poly(méth)acrylate acrylé | > 7.000 | | |

Les valeurs de températures de transition vitreuse Tg de ces oligomères (non réticulés) ont été mesurées par calorimétrie différentielle à balayage au moyen d'un appareil de DSC (Differential Scanning Calorimetry) à compensation de puissance, DSC Q2000 (TA Instruments)

Les conditions opératoires étaient les suivantes :
- Mode : standard
- Creusets porte-échantillons en aluminium hermétiques,
- Gaz de balayage : Azote de qualité U (50 ml/min)
- Rampe de température utilisée :
   Isotherme à -90 °C pendant 5 minutes
   De -90 à 200°C à 10°C/min
   Isotherme à 200°C pendant 5 minutes
   De 200 à -90°C à 20°C/min
   Isotherme à -90°C pendant 5 minutes
   De -90 à 200°C à 10 °C/min
   Isotherme à 200°C pendant 5 minutes

De manière avantageuse, la composition de vernis selon l'invention renferme une concentration en dit oligomère comprise entre 0,1 % et 30 % en poids, de préférence comprise entre 0,5 % et 20 % en poids, de préférence encore comprise entre 1 et 15 % en poids du poids total de la composition de vernis.

Avantageusement, la composition de vernis, selon l'invention, renferme une teneur en solvant comprise entre 60% et 85%. Une telle teneur en solvant permet d'avoir une composition suffisamment fluide, d'application aisée sur l'ongle.

Ce type de composition conduit à un film brillant sur l'ongle, sur une durée plus longue que les vernis-solvants classiques. Son démaquillage est aisé, ne nécessitant pas un contact prolongé avec le dissolvant.

Un premier type de composition de vernis selon l'invention, appelée première composition ou composition de base, ne renferme pas d'agent de réticulation de l'oligomère, tel qu'un photo-initiateur, elle peut donc être appliquée directement sur l'ongle, évitant ainsi tout problème de sensibilisation ou d'allergie pour l'usager.

Un second type de composition de vernis selon l'invention, utilisable en tant que composition de finition appelée aussi composition « top-coat » ou seconde composition, destinée à être appliquée sur la composition de base, comprend, en outre, un photo-initiateur, de préférence de type radicalaire, apte à amorcer la réticulation du ou des oligomères (au moins) à la lumière du jour (c'est-à-dire sans nécessiter l'utilisation d'une lampe U.V.). Ainsi le photo-initiateur n'est pas en contact direct avec l'ongle.

La concentration en photo-initiateur de ladite composition « top-coat » est, avantageusement, comprise entre 0,1 % et 10 % en poids, de préférence entre 0,5 % et 5 % en poids du poids total de la composition de vernis.

La présente invention concerne également un procédé d'application de vernis sur un ongle comprenant les étapes successives suivantes :
i) Application sur l'ongle d'une première composition A, telle que décrite ci-dessus, sous la forme d'au moins un film FA, la composition A renfermant de préférence un pigment coloré et ne renfermant pas de photo-initiateur ; suivie d'un séchage dudit film FA ;
ii)Application, à la surface du film FA formé par la composition A, d'une seconde composition B telle que décrite ci-dessus, dénommée top coat, sous la forme d'un film FB, la composition B renfermant un photo-initiateur ;
iii) Initiation, à la lumière visible, de la réticulation de la composition B, la réticulation des oligomères de la composition B provoquant la réticulation des oligomères présents à la surface du film FA, induisant une forte liaison des films FA et FB entre eux.

La présence des oligomères de la première composition A permet audit film FA un séchage rapide, et autorise ainsi sans délai l'application successive de la couche de la seconde composition B à la surface du premier film FA.

La présente invention concerne également un kit de vernis à ongles comprenant :
- un contenant CA, de type flacon, renfermant une première composition A de vernis selon l'invention, sans photo-initiateur,
- un contenant CB, de type flacon, renfermant une seconde composition B de vernis selon l'invention renfermant un photo-initiateur,
- une notice d'utilisation.

Avantageusement, le contenant CA comporte des parois au moins partiellement transparentes rendant visible la teinte du vernis coloré de la première composition A lorsque celle-ci est stockée dans ledit contenant CA et renferme un pigment coloré. L'utilisateur peut ainsi constater directement la teinte du vernis coloré.

Avantageusement, le contenant CB comporte des parois opaques à la lumière visible et aux UV, aptes à empêcher la réticulation du vernis de la seconde composition B renfermant le photo-initiateur lorsque celle-ci est stockée dans ledit contenant CB. La seconde composition B ou top-coat est généralement incolore, ou très légèrement teintée.

Le kit selon l'invention peut en outre comprendre un contenant renfermant un dissolvant apte à retirer les couches de vernis A et B de l'ongle, par exemple pour un démaquillage au moyen de cotons imprégnés dudit dissolvant.

La présente invention va maintenant être décrite plus en détail et illustrée par les exemples, non limitatifs, ci-après :

### EXEMPLES

Différentes compositions de vernis ont été testées, conformes à la présente invention et comparées à des compositions de l'art antérieur.

Tous les pourcentages indiqués ci-après sont des pourcentages massiques.

Parmi les tests réalisés sur les vernis obtenus :
- La brillance a été mesurée (sur une échelle de 100) avec un brillance-mètre Minolta 268 (angle d'incidence 60°) pour une application (100µm humide) faite sur carte type LENETA.
- Le test d'abrasion humide consiste à mesurer la résistance à l'usure causée par une brosse suite à des mouvements d'aller et retour de cette dernière sur la surface du film. Pour cela, un ou plusieurs films de 100µm humide sont appliqués sur une plaque d'aluminium. Après séchage 12h à température ambiante, la brillance de ce système est mesurée. C'est la valeur 0 aller-retour. Le système est ensuite testé et la brillance est mesurée tous les 2000 aller-retours. Plus la brillance sera élevée, plus résistant sera le film.
- La dureté a été mesurée à l'aide d'un pendule de "Persoz" sur le film sec formé par l'application d'une couche de 100 µm humide d'épaisseur de la composition sur une plaque de verre, et séché 24 h à température ambiante (25 °C), la valeur est exprimée en secondes.
- L'adhérence a été mesurée en réalisant le Cross Hatch Test sur le film sec formé par l'application d'une couche de 100 µm humide d'épaisseur de la composition sur une plaque de verre et séché une nuit à 25°C. La note 0 correspond à l'absence de perte d'adhésion. La note 5 correspond à la perte totale d'adhésion.

### Exemple comparatif 1 : Vernis-solvant classique

Ce vernis a été obtenu par application de deux couches de composition colorée et une couche de « top-coat ». Les compositions respectives étaient les suivantes :

**Composition colorée :**

| | |
|---|---|
| Acétate de butyle | 38 |
| Acétate d'éthyle | 20,7 |
| Nitrocellulose | 18 |
| Acétyl tributyl citrate | 9 |
| Copolymère acide adipique/néopentylglycol/anhydride triméllitique | 12 |
| Stearalkonium bentonite | 1,5 |
| Pigment Red 7 Lake | 0,8 |
| **TOTAL** | **100** |

**Composition de top-coat :**

| | |
|---|---|
| Acétate de butyle | 20 |
| Acétate d'éthyle | 46 |
| Acétate butyrate de cellulose | 16 |
| Alcool isopropylique | 10 |
| Copolymère acide adipique/néopentylglycol/anhydride triméllitique | 5 |
| Acétyl tributyl citrate | 3 |
| **TOTAL** | **100** |

Ce vernis présente un « séchage physique », c'est-à-dire que l'on note un séchage par évaporation du solvant et un durcissement à l'air et à la lumière visible du vernis après quelques minutes.

### Exemple comparatif 2 : Vernis-gel de l'art antérieur

Ce vernis gel commercialisé sous le nom de SHELLAC de la société Creative Nail Design comprend un ensemble de trois compositions : une composition de base, appliquée en une couche unique directement sur l'ongle et réticulée sous UV, deux couches d'une composition colorée réticulées sous UV et une couche « top-coat » d'une composition finale couvrant les couches précédentes, également réticulée sous UV. Ces trois compositions renferment une proportion importante (entre 40 et 60 %) d'un oligomère de type polyuréthane acrylate tel que le Di Hema triméthylhexyl dicarbamate de masse molaire 470 g/mol, en présence d'autres (méth)acrylates de masse molaire inférieure à 1000 g/mol (WO2011/011304). Aucun de ces composés ne présente un « séchage physique » au sens de la définition donnée en introduction. En revanche ces composés acrylés polymérisent rapidement sous un rayonnement ultra-violet pour former un film résistant sur l'ongle. Il est donc indispensable de disposer d'une lampe UV pour réaliser successivement les polymérisations/réticulations des différentes couches appliquées.

### Exemple 3 : invention

Ce vernis comprend une composition colorée et une composition de finition (top-coat), dont les formulations sont présentées ci-après :

**Composition colorée (dénommée 1ere composition):**

| | |
|---|---|
| Acétate de butyle | 31 |
| Acétate d'éthyle | 31,2 |
| Nitrocellulose | 15 |
| Acétyl tributyl citrate | 6 |
| Copolymère acide adipique/néopentylglycol/anhydride triméllitique | 12 |
| Oligomère | 2,5 |
| Stearalkonium bentonite | 1,5 |
| Pigment Red 7 Lake | 0,8 |
| **TOTAL** | **100** |

**Composition de finition (dénommée 2^{e} composition) :**

| | |
|---|---|
| Acétate de butyle | 19,4 |
| Acétate d'éthyle | 46 |
| Acétate butyrate de cellulose | 15 |
| Alcool isopropylique | 10 |
| Photoinitiateur | 2 |
| Acétyl tributyl citrate | 2,6 |
| Oligomère | 5 |
| **TOTAL** | **100** |

Dans cet exemple, le photoinitiateur est l'éthyl (-2,4,6,-trimethylbenzoyl) phenylphosphinate (TPO I) de la société Rahn, l'oligomère, le produit Doublemer 347-1 de la société Double Bound et le pigment Red 7 Lake, Unipur Red LC 3071 de la société Sensient.

Au contraire du vernis Shellac de l'exemple comparatif 2, la 1ere composition, celle au contact direct de l'ongle, ne comprend pas de photo-initiateur, mais sèche sur l'ongle en quelques minutes, autorisant son recouvrement par la composition de finition. La composition de finition renferme un photo-initiateur pour accélérer et réaliser une polymérisation/réticulation complète de l'oligomère à la lumière visible, sans nécessiter l'usage d'une lampe UV.

Des tests de brillance ont été réalisés sur ce vernis, ainsi que les vernis des exemples comparatifs 1 et 2. Pour l'exemple 2, le vernis comparatif a été passé sous lampe UV de 36 Watt après application de la composition de base (10 secondes), de la première couche colorée (2 minutes), de la seconde couche colorée (2 minutes) et de la couche "top coat" (4 minutes). L'ensemble a été ensuite dégraissé à l'isopropanol comme indiqué dans la notice du fabriquant.

### Brillance à t₀

Exemple 1 (comp.)= 82 UB
Exemple 2 (comp.)= 85 UB
Exemple 3 = 88 UB

Les résultats de la perte de brillance en % en fonction du nombre de passages lors du test d'abrasion humide sont regroupés dans le tableau 2 ci-après.

**Tableau 2**

| | Vernis 1 (Comp.) | Vernis 2 (Comp.) | Vernis 3 (invention) |
|---|---|---|---|
| 0 passe | 100 | 100 | 100 |
| 2 000 passes | 99,1 | 100 | 100 |
| 4 000 passes | 98,8 | 99,6 | 99,5 |
| 6 000 passes | 97,6 | 99,3 | 99,3 |
| 8 000 passes | 96,3 | 99 | 99,1 |
| 10 000 passes | 96,3 | 98,7 | 98,8 |

La perte de brillance du vernis selon l'invention est minime, du même ordre de grandeur que celle du vernis-gel.

Les temps de maquillage et de démaquillage ont également été notés, ils sont regroupés dans le tableau 3 ci-après.

**Tableau 3**

| **Vernis** | **Temps de maquillage** | **Temps de démaquillage** |
|---|---|---|
| Ex. 1 (Comp.) | 5 minutes | 5 minutes avec dissolvant base acétate éthyle |
| Ex. 2 (Comp.) | 25 minutes | 30 minutes avec dissolvant base acétone |
| Ex. 3 | 5 minutes | 5 minutes avec dissolvant base acétate éthyle |

On note un maquillage et un démaquillage du vernis selon l'invention équivalent à celui d'un vernis-solvant classique, et bien inférieurs à ceux du vernis-gel, sans nécessiter l'emploi d'un solvant à base d'acétone.

Un test a été réalisé sur un panel de 20 personnes. Un premier groupe a testé le vernis 1, un second groupe le vernis 2 et un troisième groupe le vernis 3. Ces panelistes ont donné leur avis sur la tenue de la brillance du système appliqué. La moyenne des réponses est présentée dans le tableau 4 ci-dessous.

**Tableau 4**

| **Vernis** | **Tenue de la brillance** |
|---|---|
| Vernis 1 (Comp.) | 4 jours |
| Vernis 2 (Comp.) | 10 jours |
| Vernis 3 | 8 jours |

On note une tenue de la brillance doublée par rapport au vernis-solvant classique.

Des mesures de dureté (Persoz) ont également été effectuées sur les vernis des exemples 1, 2 et 3 après séchage :
- sur le vernis de base (vernis coloré formé par l'application de deux couches de 100 µm humides)
- sur le vernis de base précédent sur lequel a été appliquée une couche de "top coat" de 100 µm humide.

Les résultats sont regroupés dans le tableau 5 ci-dessous.

**Tableau 5**

| | 2 couches de vernis coloré | 2 couches de vernis coloré + 1 couche de top coat |
|---|---|---|
| Ex. 1 (comp.) | 242 s | 226 s |
| Ex. 2 (comp.) | 36 s | 25 s |
| Ex. 3 (invention) | 267 s | 251 s |

On note une dureté supérieure pour le vernis selon l'invention avant et après application de la couche de finition (top coat) même en l'absence de photo-initiateur pour les couches de vernis coloré.

### Exemple 4 : proportions d'oligomère et de photo-initiateur

Dans cet exemple, différents pourcentages d'oligomère et de photo-initiateur ont été introduits dans la composition de finition (top-coat) appliquée sur le même vernis « coloré » que celui de l'exemple 3. Les trois compositions de finition supplémentaires testées sont regroupées dans le tableau 6.

**Tableau 6**

| | **Vernis 3 de l'ex. 3** | **Vernis 4** | **Vernis 5** | **Vernis 6** |
|---|---|---|---|---|
| Acétate de butyle | 19,4 | 20,4 | 17,4 | 19,4 |
| Acétate d'éthyle | 46 | 48 | 43 | 44 |
| Acétate butyrate de cellulose | 15 | 15 | 15 | 15 |
| Alcool isopropylique | 10 | 10 | 10 | 10 |
| Photoinitiateur | 2 | 2 | 2 | 4 |
| Acétyl tributyl citrate | 2,6 | 2,6 | 2,6 | 2,6 |
| Oligomère | 5 | 2 | 10 | 5 |
| **TOTAL** | **100** | **100** | **100** | **100** |

Le photoinitiateur est l'éthyl (2,4,6,-trimethylbenzoyl) phenylphosphinate (TPO I) et l'oligomère, le produit Doublemer 347-1 de la société Double Bound.

De manière similaire à l'exemple 3 des mesures de brillance, de perte de brillance dans un test d'abrasion humide (tableau 7), de temps de maquillage et de démaquillage (tableau 8) ont été effectués, et sont présentés ci-après.

### Brillance à t₀

Vernis 3 = 88 UB
Vernis 4 = 86 UB
Vernis 5 = 89 UB
Vernis 6 = 87 UB

Les résultats de la perte de brillance en % en fonction du nombre de passages lors du test d'abrasion humide sont regroupés dans le tableau 7 ci-après.

**Tableau 7**

| | **Vernis 3** | **Vernis 4** | **Vernis 5** | **Vernis 6** |
|---|---|---|---|---|
| **0 passe** | 100 | 100 | 100 | 100 |
| **2 000 passes** | 100 | 99,7 | 100 | 100 |
| **4 000 passes** | 99,5 | 99,1 | 99,6 | 99,4 |
| **6 000 passes** | 99,3 | 98,5 | 99,5 | 99,4 |
| **8 000 passes** | 99,1 | 98,1 | 99,2 | 99,0 |
| **10 000 passes** | 98,8 | 97,7 | 98,9 | 98,7 |

**Tableau 8**

| **Vernis** | **Temps de maquillage** | **Temps de démaquillage** |
|---|---|---|
| Vernis 3 | 5 minutes | 5 minutes avec dissolvant base acétate éthyle |
| Vernis 4 | 5 minutes | 5 minutes avec dissolvant base acétate éthyle |
| Vernis 5 | 5 minutes | 7 minutes avec dissolvant base acétate éthyle |
| Vernis 6 | 5 minutes | 6 minutes avec dissolvant base acétate éthyle |

## Revendications

1. Composition de vernis pour les ongles, renfermant au moins un agent filmogène, un plastifiant et un solvant,
**caractérisée en ce qu'**elle comprend également un ou plusieurs oligomère(s) de masse molaire supérieure à 1500 g/mol et/ou dont la température de transition vitreuse Tg est supérieure à 25 °C, ledit oligomère possédant des doubles liaisons acryliques ou méthacryliques disponibles pour réagir avec des radicaux, sans renfermer d'agent de réticulation de l'oligomère, tel qu'un photo-initiateur.

2. Composition de vernis selon la revendication 1,
**caractérisée en ce que** la masse molaire de l'oligomère est supérieure ou égale à 2000 g/mol, de préférence supérieure ou égale à 3000 g/mol.

3. Composition de vernis selon la revendication 1 ou 2,
**caractérisée en ce que** l'oligomère présente une température de transition vitreuse Tg supérieure à 30 °C, de préférence supérieure à 35 °C, de préférence encore supérieure à 40°C.

4. Composition de vernis selon l'une quelconque des revendications précédentes,
**caractérisée en ce que** l'oligomère présente une dureté Persoz d'au moins 60 s, de préférence d'au moins 80 s, de préférence encore d'au moins 100 s, lorsqu'il est appliqué sur une plaque de verre selon une épaisseur de 100 µm humide, puis séché 24 h à 25°C à l'abri de la lumière.

5. Composition de vernis selon l'une quelconque des revendications précédentes,
**caractérisée en ce que** l'oligomère est choisi parmi : un poly(méth)acrylate (meth)acrylé, un polyester (meth)acrylé, un polyether (meth)acrylé, un uréthane (meth)acrylé, un polyurethane (meth)acrylé ou un époxy (meth)acrylé, un poly(méth)acrylate modifié silane (méth)acrylé ou un mélange de ceux-ci.

6. Composition de vernis selon l'une quelconque des revendications précédentes,
**caractérisée en ce qu'**elle renferme une concentration en dit oligomère comprise entre 0,1 % et 30 % en poids, de préférence comprise entre 0,5 % et 20 % en poids, de préférence encore comprise entre 1 et 15 % en poids du poids total de la composition de vernis.

7. Procédé d'application de vernis sur un ongle comprenant les étapes successives suivantes :
i)Application sur l'ongle d'une première composition A conforme à l'une quelconque des revendications 1 à 6 sous la forme d'au moins un film FA, la composition A renfermant de préférence un pigment coloré et ne renfermant pas de photo-initiateur ; suivie d'un séchage dudit film FA ;
ii) application, à la surface du film FA formé par la composition A, d'une seconde composition B, dénommée top coat, conforme à l'une quelconque des revendications 1 à 6 et comprenant de plus un photo-initiateur, sous la forme d'un film FB ;
iii) initiation, à la lumière visible, de la réticulation de la composition B, la réticulation des oligomères de la composition B provoquant la réticulation des oligomères présents à la surface du film FA, induisant une forte liaison des films FA et FB entre eux.

8. Kit de vernis à ongles,
**caractérisé en ce qu'**il comprend :
- un contenant CA, de type flacon, renfermant une première composition A de vernis conforme à l'une quelconque des revendications 1 à 6,
- un contenant CB, de type flacon, renfermant une seconde composition B de vernis conforme à l'une quelconque des revendications 1 à 6 comprenant de plus un photo-initiateur,
- une notice d'utilisation.

9. Kit selon la revendication 8,
**caractérisé en ce que** le photo-initiateur de la composition B est de type radicalaire apte à amorcer la réticulation du ou des oligomères (au moins) à la lumière du jour.

10. Kit selon la revendication 8 ou 9,
**caractérisé en ce que** la concentration en photo-initiateur est comprise entre 0,1 % et 10 % en poids, de préférence entre 0,5 % et 5 % en poids du poids total de la composition de vernis.

11. Kit selon l'une quelconque des revendications 8 à 10,
**caractérisé en ce que** le contenant CA comporte des parois au moins partiellement transparentes rendant visible la teinte du vernis coloré de la première composition A, lorsque celle-ci est stockée dans ledit contenant CA.

12. Kit selon l'une quelconque des revendications 8 à 11,
**caractérisé en ce que** le contenant CB comporte des parois opaques à la lumière visible et aux UV, aptes à empêcher la réticulation du vernis de la seconde composition B renfermant le photo-initiateur, lorsque celle-ci est stockée dans ledit contenant CB.

13. Kit selon l'une quelconque des revendications 8 à 12,
**caractérisé en ce qu'**il comprend un contenant renfermant un dissolvant apte à retirer les couches de vernis A et B de l'ongle.

## Patentansprüche

1. Lackzusammensetzung für die Nägel, einschließend mindestens ein filmbildendes Mittel, einen Weichmacher und ein Lösungsmittel,
**dadurch gekennzeichnet, dass** sie ebenfalls ein oder mehrere Oligomer(e) mit einer molaren Masse über 1500 g/mol umfasst und/oder deren Glasübergangstemperatur Tg über 25 °C beträgt, wobei das Oligomer verfügbare Acryl- oder Methacryl-Doppelbindungen besitzt, um mit Radikalen zu reagieren, ohne das Vernetzungsmittel des Oligomers wie einen Photoinitiator einzuschließen.

2. Lackzusammensetzung nach Anspruch 1,
**dadurch gekennzeichnet, dass** die molare Masse des Oligomers größer oder gleich 2000 g/mol, vorzugsweise größer oder gleich 3000 g/mol, ist.

3. Lackzusammensetzung nach Anspruch 1 oder 2,
**dadurch gekennzeichnet, dass** das Oligomer eine Glasübergangstemperatur Tg über 30 °C, vorzugsweise über 35 °C, vorzugsweise noch über 40 °C, aufweist.

4. Lackzusammensetzung nach einem der vorangehenden Ansprüche,
**dadurch gekennzeichnet, dass** das Oligomer eine Persoz-Härze von mindestens 60 s, vorzugsweise von mindestens 80 s, vorzugsweise noch von mindestens 100 s, aufweist, wenn es auf eine Glasplatte in einer Stärke von 100 µm feucht aufgetragen, dann 24 Stunden bei 25 °C vor Licht geschützt getrocknet wird.

5. Lackzusammensetzung nach einem der vorangehenden Ansprüche,
**dadurch gekennzeichnet, dass** das Oligomer ausgewählt ist aus: einem (meth)acrylierten Poly(meth)acrylat, einem (meth)acrylierten Polyester, einem (meth)acrylierten Polyether, einem (meth)acrylierten Urethan, einem (meth)acrylierten Polyurethan oder einem (meth)acrylierten Epoxy, einem (meth)acrylierten silanmodifizierten Poly(meth)acrylat oder einem Gemisch derselben.

6. Lackzusammensetzung nach einem der vorangehenden Ansprüche,
**dadurch gekennzeichnet, dass** sie eine Konzentration an besagtem Oligomer einschließt, die zwischen 0,1 und 30 Gew.-%, vorzugsweise zwischen 0,5 und 20 Gew.-%, vorzugsweise noch zwischen 1 und 15 Gew.-% des Gesamtgewichts der Lackzusammensetzung liegt.

7. Verfahren zum Auftragen von Lack auf einen Nagel, umfassend die folgenden aufeinanderfolgenden Schritte:
i) Auftragen, auf den Nagel, einer ersten Zusammensetzung A nach einem der Ansprüche 1 bis 6 in Form von mindestens einem Film FA, wobei die Zusammensetzung A vorzugsweise ein farbiges Pigment einschließt und keinen Photoinitiator einschließt; gefolgt von einem Trocknen des Films FA;
ii) Auftragen, auf die Oberfläche des Films FA, gebildet von der Zusammensetzung A, einer zweiten Zusammensetzung B, bezeichnet als Top Coat, nach einem der Ansprüche 1 bis 6 und umfassend zusätzlich einen Photoinitiator in Form eines Films FB;
iii) Initiieren, unter sichtbarem Licht, der Vernetzung der Zusammensetzung B, wobei die Vernetzung der Oligomere der Zusammensetzung B die Vernetzung der Oligomere bewirkt, die auf der Oberfläche des Films FA vorhanden sind, was eine starke Verbindung der Filme FA und FB untereinander induziert.

8. Nagellackset,
**dadurch gekennzeichnet, dass** es umfasst:
- ein Behältnis CA vom Typ Flakon, das eine erste Lackzusammensetzung A nach einem der Ansprüche 1 bis 6 einschließt,
- ein Behältnis CB vom Typ Flakon, das eine zweite Lackzusammensetzung B nach einem der Ansprüche 1 bis 6 einschließt, umfassend zusätzlich einen Photoinitiator,
- eine Gebrauchsanweisung.

9. Set nach Anspruch 8,
**dadurch gekennzeichnet, dass** der Photoinitiator der Zusammensetzung B vom Radikaltyp ist, der imstande ist, die Vernetzung des oder der Oligomer (mindestens) bei Tageslicht zu starten.

10. Set nach Anspruch 8 oder 9,
**dadurch gekennzeichnet, dass** die Konzentration an Photoinitiator zwischen 0,1 und 10 Gew.-%, vorzugsweise zwischen 0,5 und 5 Gew.-% des Gesamtgewichts der Lackzusammensetzung liegt.

11. Set nach einem der Ansprüche 8 bis 10,
**dadurch gekennzeichnet, dass** das Behältnis CA mindestens teilweise transparente Wände aufweist, die die Farbe des Farblacks der ersten Zusammensetzung A sichtbar machen, wenn sie in dem Behältnis CA gelagert ist.

12. Set nach einem der Ansprüche 8 bis 11,
**dadurch gekennzeichnet, dass** das Behältnis CB für das sichtbare Licht und für UV opake Wände aufweist, die imstande sind, die Vernetzung des Lacks der zweiten Zusammensetzung B, welche den Photoinitiator einschließt, zu verhindern, wenn diese in dem Behältnis CB gelagert ist.

13. Set nach einem der Ansprüche 8 bis 12,
**dadurch gekennzeichnet, dass** es ein Behältnis umfasst, das einen Entferner einschließt, der imstande ist, die Lackschichten A und B von dem Nagel zu entfernen.

## Claims

1. Nail varnish composition containing at least one film-forming agent, a plasticizer and a solvent,
**characterized in that** it also comprises one or more oligomers with a molar mass greater than 1500 g/mol and/or whose glass transition temperature Tg is greater than 25°C, wherein the oligomer comprises acrylic or methacrylic double bonds to react with radicals, without containing an oligomer crosslinking agent, such as a photo-initiator.

2. Nail varnish composition according to claim 1,
**characterized in that** the molar mass of the oligomer is greater than or equal to 2000 g/mol, preferably greater than or equal to 3000 g/mol.

3. Nail varnish composition according to claim 1 or 2,
**characterized in that** the oligomer has a glass transition temperature Tg greater than 30°C, preferably greater than 35°C, more preferably greater than 40°C.

4. Nail varnish composition according to any one of the preceding claims,
**characterized in that** the oligomer has a Persoz hardness of at least 60 s, preferably at least 80 s, more preferably at least 100 s, when applied to a glass plate in accordance with a thickness of 100 µm wet, then dried 24 hours at 25°C away from light.

5. Nail varnish composition according to any one of the preceding claims,
**characterized in that** the oligomer is selected from among: a (meth)acrylated poly(meth)acrylate, a (meth)acrylated polyester, a (meth)acrylated polyether, a (meth)acrylated urethane, a (meth)acrylated polyurethane or a (meth)acrylated epoxy, a modified silane (meth)acrylated poly(meth)acrylate, or a mixture thereof.

6. Nail varnish composition according to any one of the preceding claims, **characterized in that** it contains a concentration of the said oligomer of between 0.1% and 30% by weight, preferably between 0.5% and 20% by weight, more preferably between 1 and 15% by weight relative to the total weight of the nail varnish composition.

7. Method of applying varnish to a nail comprising the following successive steps:
i) Application to the nail of a first composition A according to any one of the claims 1 to 6 in the form of at least one film FA, wherein the composition A preferably contains a colored pigment and does not contain a photo-initiator; followed by drying the FA film;
ii) Application to the surface of the film FA formed by the composition A, of a second composition B, referred to as the top coat, according to any one of claims 1 to 6, and further comprising a photo-initiator, in the form a film FB.
iii) Initiation, in visible light, of the crosslinking of the composition B, wherein the crosslinking of the oligomers of the composition B causes the crosslinking of the oligomers present on the surface of the film FA, inducing a strong bond of the FA and FB films together.

8. Nail varnish kit,
**characterized in that** it comprises:
- a bottle-type container CA containing a first varnish composition A according to any one of the claims 1 to 6,
- a bottle-type container CB containing a second varnish composition B according to any one of the claims 1 to 6 further comprising a photo-initiator,
- user instructions.

9. Kit according to claim 8,
**characterized in that** the photo-initiator of the composition B is of a radical type capable of initiating the crosslinking of the oligomer(s) (at least) in the light of day.

10. Kit according to claim 8 or 9,
**characterized in that** the photo-initiator concentration is between 0.1% and 10% by weight, preferably between 0.5% and 5% by weight of the total weight of the varnish composition.

11. Kit according to any one of the claims 8 to 10,
**characterized in that** the container CA has at least partially transparent walls rendering visible the hue of the colored varnish of the first composition A, when it is stored in the CA container.

12. Kit according to any one of the claims 8 to 11,
**characterized in that** the container CB has walls that are opaque to visible light and UV in order to prevent crosslinking of the varnish of the second composition B containing the photo-initiator, when it is stored in the container CB.

13. Kit according to any one of the claims 8 to 12,
**characterized in that** it comprises a container containing a solvent capable of removing the layers of varnish A and B from the nail.
